# EUROPEAN PATENT SPECIFICATION

(11) **EP 4 648 829 B1**
(45) Date of publication and mention of the grant of the patent: **19.08.2026**
(21) Application number: 24705563.5
(22) Date of filing: 24.01.2024
(51) Int. Cl.: A61M 15/00, A61M 11/00, B05B 11/00

(54) **A CARTRIDGE FOR AN INHALER**
KARTUSCHE FÜR EINEN INHALATOR
CARTOUCHE POUR INHALATEUR

(30) Priority: 24.01.2023 GB 202301009
(43) Date of publication of application: 19.11.2025
(73) Proprietor: Merxin Ltd, King's Lynn, Norfolk PE30 5FQ (GB)
(72) Inventor: PURKINS, Graham, Leziate King's Lynn PE32 1EN (GB); HODSON, Peter David, Derby, DE72 3EL (GB); STUART, Adam, Pott Row King's Lynn PE32 1BS (GB); ANTONIAK, Dylan, King's Lynn PE30 5GE (GB); BARRETT, Adam, West Winch King's Lynn PE33 0PR (GB)
(74) Representative: Basck Limited
(86) International application number: PCT/IB2024/050662
(87) International publication number: WO 2024/157182

(56) References cited:
- WO-A2-00/49988
- US-A1- 2017 203 056
- US-A1- 2020 179 620
- US-B2- 10 576 222
- US-B2- 10 603 451
- US-B2- 10 973 997

## Description

### FIELD OF THE INVENTION

The present invention relates to a cartridge for use in an inhaler. More particularly, the present invention relates to a cartridge for use in an inhaler that dispenses liquid medicament. The present invention also relates to an inhaler for dispensing liquid medicament that comprises a cartridge.

### BACKGROUND

In recent years, the use of inhalers for delivering medication for treating diseases related to lungs, respiration, and the like has increased. Inhalers used for this purpose are typically capable of dispensing small quantities of liquids containing dissolved pharmaceutical compounds to a user in a controlled and accurate manner. This type of inhaler often includes a cartridge containing a solution of a liquid medicament, the inhaler configured to release the liquid medicament in the form of a mist during actuation of the inhaler.

Typically, this type of cartridge is designed to protect its contents during long-term storage. However, over time a portion of the liquid may gradually be lost from the cartridge due to diffusion or evaporation, and the concentration level of the dissolved pharmaceutical compound may therefore increase to an unacceptable level. Moreover, this effect may be significantly more pronounced once the seal of the cartridge has been broken for its first use. Therefore, cartridges can only generally be used for a relatively short period of time once the seal has been broken before they need to be discarded.

Conventionally, in order to increase the shelf life of the cartridges, diffusion or evaporation is reduced by the use of non-permeable or low-permeability materials for seals and cartridge structure, for example aluminium foil. However, once a cartridge is inserted into an inhaler and used for the first time, the aluminium foil seal is broken and the contents of the cartridge is therefore susceptible to diffusion and evaporation of the liquid contents. Significant changes in drug formulation concentration can occur through changes in the volume of the liquid as a consequence of diffusion or evaporation. This hampers the dose uniformity of the liquid medicament dispensed by the inhaler.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

US10973997B2 discloses a nebulizer for nebulizing a liquid from a container is proposed. The nebulizer comprises a liquid pump for withdrawing the liquid in doses from the container and pressurizing the respective doses for nebulization. The nebulizer comprises in addition an air pump for temporarily pressurizing the liquid in the container to help withdrawing the liquid from a collapsible bag in the container.

US2020/179620A1 discloses a nebulizer for nebulizing a liquid from a container, where the nebulizer includes a fluid pump for withdrawing the liquid in doses from the container and pressurizing the respective doses for nebulization. The container includes an air pump with a piston/cylinder arrangement to pressurizing the liquid in the container to help withdrawing the liquid from the container. A control valve limits the air pressure acting on the liquid.

US10603451B2 discloses a container for holding, dispensing and/or storing a preferably liquid medicament preparation is proposed, which comprises an inner space for the medicament preparation and an at least partially multi-layered wall structure defining the inner space, the wall structure comprising a first layer with a first through-opening of less than 40 µm. Alternatively or additionally, the wall structure comprises a second and third layer, a second through-opening being provided in the second layer and the third layer covering or closing off the wall structure.

WO00/49988A2 discloses a diffusion-tight container which is capable of being stored over long periods of time and with which the liquid does not come into contact with the ambient air is required for withdrawing a liquid in a dosed manner out of the same over a duration of several months. The cartridge is a container which is comprised of three shells and which can consist of a collapsible bag containing the liquid, of a dimensionally stabile container, and of a stiff metal shell. The cartridge can be detachably connected to a withdrawing device. The cartridge can be provided with a micro-opening with which the time for the pressure compensation between the cartridge and the surroundings can be adjusted. The cartridge is suited for aqueous and alcoholic liquids which contain a pharmacologically active ingredient. The liquid in the cartridge is protected against external influences. The cartridge can be used in an atomizer for generating an aerosol which can be inhaled and which is provided for treating illnesses.

US 2017/203056A1 discloses a drug delivery device, in particular a nebulizer or inhaler, wherein a collapsible container is combined with a mechanism to help the collapsing process and to prevent the collapsed container expanding again, hence preventing forming of vapor and gas bubbles within.

US10576222B2 discloses a nebulizer as well as a container and an indicator device for such a nebulizer are proposed. The indicator device is fixedly mounted from the bottom of the container and comprises a piercing element for opening an aeration of the container. The indicator device and its piercing part are preferably actuated by axial movement of the indicator device and container within the nebulizer.

### SUMMARY OF THE INVENTION

It is an object of the present invention to provide a cartridge for an inhaler which goes some way to overcoming the abovementioned disadvantages or which at least provides the public or industry with a useful choice. The present invention provides therefore a cartridge for an inhaler as defined in claim 1, and an inhaler for delivery of liquid medicament to a user as defined in claim 10.

The term "comprising" as used in this specification and indicative independent claims means "consisting at least in part of". When interpreting each statement in this specification and indicative independent claims that includes the term "comprising", features other than that or those prefaced by the term may also be present. Related terms such as "comprise" and "comprises" are to be interpreted in the same manner.

As used herein the term "and/or" means "and" or "or", or both.

As used herein "(s)" following a noun means the plural and/or singular forms of the noun. Accordingly, in a first aspect the present invention may broadly be said to consist in a cartridge **for an inhaler,** comprising: an inner cartridge assembly comprising a bottle and a collapsible bag, the bag configured to contain a volume of liquid, the bottle and bag mutually configured so that the bag is contained within the bottle, the bottle further comprising a container hole configured to allow gases to pass between the interior and exterior of the bottle; an outer casing configured to contain the inner cartridge assembly, the inner cartridge assembly sealed within the outer casing, the outer casing comprising a vent hole passing through the casing from the exterior to the interior to allow gases to pass into the outer casing; a sealing assembly configured to close and seal the vent hole, the sealing assembly having an open position that allows the passage of gases through the vent hole, and a closed position where the sealing assembly seals the vent hole, the sealing assembly configured so that when the cartridge is used in an inhaler, actuating the inhaler temporarily opens the sealing assembly to allow the passage of gases therethrough; the outer casing and inner cartridge assembly further configured so that there is a gas path inside the cartridge between the container hole and the vent hole.

In an embodiment, the sealing assembly comprises a spring and a bung, the bung and vent hole mutually configured so that the bung can locate onto the vent hole to seal the vent hole, the spring configured to bias the bung towards the vent hole.

In an embodiment, the spring comprises a conical spring.

In an embodiment, the spring is configured so as to taper towards the bung.

In an embodiment, the spring comprises a finger disc spring.

In an embodiment, the bung comprises a rounded outer end.

In an embodiment, the bung comprises a main body portion and an insert that locates into a cavity in the main body portion, the insert having a greater width or diameter than that of the vent hole, the main body portion formed from a material that retains shape when subjected to compressive forces in use, the insert formed from a sealing material softer than the material used for the main body portion.

In an embodiment, the spring is configured so as to taper away from the bung.

In an embodiment, the cartridge assembly further comprises a pierceable sealing element configured to extend across and close the vent hole.

In a second aspect the present invention may broadly be said to consist in **an inhaler** for delivery of liquid medicament to a user, the inhaler comprising: a body containing the cartridge of any one of the preceding statements; the body further comprising a protrusion configured to interact with the sealing assembly in use to temporarily open the sealing assembly during actuation of the inhaler.

In an embodiment, the inhaler comprises a Soft Mist Inhaler.

With respect to the above description then, it is to be realised that the optimum dimensional relationships for the parts of the invention, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present invention, the scope of which is defined by the appended claims.

Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the invention, which is defined by the appended claims.

Throughout the description and claims of this specification, the words *"comprise", "include", "have",* and *"contain"* and variations of these words, for example *"comprising"* and *"comprises",* mean *"including but not limited to",* and do not exclude other components, items, integers or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

### BRIEF DESCRIPTION OF THE DRAWINGS

One or more embodiments of the invention will now be described, by way of example only, with reference to the accompanying drawings, in which:
**Figure 1** shows an exploded side view of the inner components or inner cartridge assembly of a cartridge in accordance with a first embodiment of the invention;
**Figure 2** shows a cross sectional side view of a cartridge according to an embodiment of the invention, the cartridge including the inner components of figure 1, the cartridge in use with a dispensing device;
**Figure 3** shows a cross-sectional side view of a cartridge in accordance with a second embodiment of the invention, the cartridge in use with a dispensing device; and
**Figure 4** shows a cross sectional side view of a cartridge in accordance with a third embodiment of the invention, the cartridge in use with a dispensing device.

### DETAILED DESCRIPTION

For all of the embodiments described below, similar numbering is used for the same or similar features - e.g. bottle 101, 201, 301; outer cartridge casing 102, 202, 302, etc. It should also be noted that where directional indicators are used - top, bottom, etc - there are intended to refer to the apparatus in the orientation shown in the figures, and are not intended to be absolute.

### Cartridge - First Embodiment

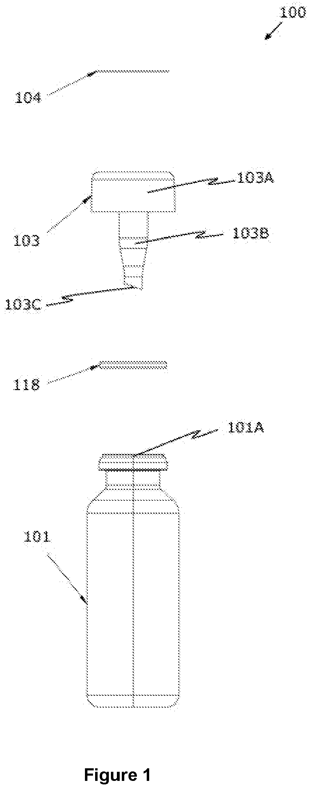

Figure 1 shows an inner cartridge assembly 100 in accordance with a first embodiment of the invention, the inner cartridge assembly 100 comprising main parts as follows: a bottle 101; a gasket 118; a closure 103, and; a pierceable seal 104.

The bottle 101 is in the preferred embodiment formed from plastic and has a top opening 101A. The opening 101A allows the bottle 101 to be filled with a liquid during assembly of the cartridge 100. The top of the bottle 101 is substantially circular in plan view, with a short neck tapering from the main body of the bottle to a lip that runs around the opening 101A. The lip and opening are circular in plan view and are smaller in diameter than the width or diameter of the main body of the bottle 101, so the outer circumferential perimeter of the lip or top of the bottle is fully enclosed within the perimeter of the main body in plan view.

The gasket 118 is formed from an elastomer material, such as for example silicone. However, in variations this could be formed from TPEs or TPVs and comprises a hollow ring with an outer diameter substantially the same diameter as the top of the bottle 101, and an inner diameter substantially the same as the opening 101A. That is, it will fit substantially exactly on/over the circular top of the bottle 100. In use, the gasket 118 is located on the open top 101A of the bottle, with the conical tube 103B of the closure 103 (described below) passing through the open centre of the gasket 118 and opening 101A, and into the top end of the bottle.

The closure 103 comprises an upper cap portion 103A and lower conical tube 103B. The closure 103 is in the preferred embodiment formed as a unitary item from a single piece of polypropylene. The closure 103 appears circular in plan view (that is, the upper cap portion 103A is circular in plan view), and has a generally capital-T-shaped appearance in side view, the upper cap portion 103A overhanging the narrower lower conical tube 103B.

The conical tube 103B tapers from the top end towards the bottom end (so that it appears conical in side view), and is sized so that the tube 103B can easily fit into the open top 101A of the bottle 100. In this embodiment, the conical tube 103B has an open lower end 103C (that end opposite the cap portion 103A). In variations of this embodiment, the lower end 103C is covered with a second pierceable seal, to further reduce vapour transmission through the closure 103.

The cap portion 103A and the top of the bottle 101 around the opening 101A are mutually configured to engage with one another in use. That is, the edge of the cap portion 103A engages with the lip around the opening 101A, overhanging and engaging around the lip.

The pierceable seal 104 comprises a thin layer of polyethylene terephthalate (PET), and is configured to cover the top surface of the cap portion 103A of the closure 103, the seal 104 circular in plan view and having substantially the same diameter as the top of the cap portion 103A.

During assembly of the inner cartridge assembly 100, the closure 103 engages with the top of the bottle 101, so that the cap portion 103A and the seal 104 (and the seal at the open end 103C if present) close the opening 101A, and the conical tube 103B is inserted into the top of the bottle 101 through the gasket 118 and opening 101A. The gasket 118 becomes sandwiched between the cap 103A and the bottle 101 to provide a seal therebetween. The pierceable seal 104 and gasket 118 together act to reduce vapour transmission from the closure 103.

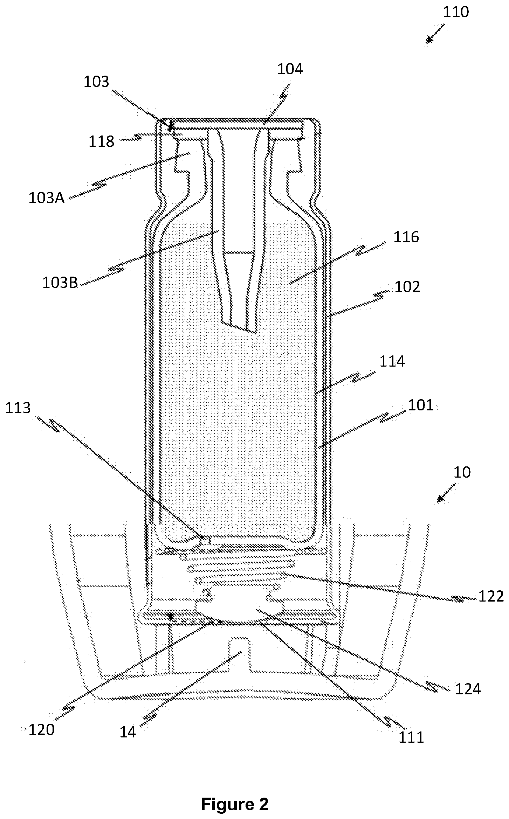

The inner cartridge assembly 100 further comprises a collapsible bag 114 (shown in figure 2, but not shown in figure 1) that forms an inner lining within the bottle 101. The collapsible bag 114 is in fluid communication with the closure 103. In use, the bottle 101 is filled with a liquid 116, with the liquid located in the bag 114. The liquid 116 comprises a solution of an active pharmaceutical agent (API) dissolved in water.

The gasket 118 is arranged between the pierceable seal 104 and the bottle 101 so as to reduce evaporation of the liquid 116 from the collapsible bag 114.

Figure 2 shows the inner cartridge assembly 100 located within an outer cartridge casing 102 or outer bottle 102. The outer casing 102 is formed as an aluminium container. The outer casing 102 and cartridge assembly 100 are mutually configured so that the inner cartridge assembly 100 fits snugly within the outer casing 102. Together, the inner cartridge assembly 100 and the outer cartridge casing 102 form a complete cartridge or cartridge assembly 110.

As shown in figure 2, the base or bottom of the inner cartridge assembly 100 has a hole 113 (container hole 113), with this hole 113 extending through the bottle 101, so that pressure changes that occur within the outer bottle 102 are transmitted to the interior of the bottle 101 The hole does not pass through into the bag 114. The container hole 113 in the preferred embodiment has an area of about 20mm² or less.

The outer cartridge casing 102 has an opening or vent hole 111 that passes through the base or bottom of the outer cartridge casing 102. The cartridge assembly 110 further comprises a foil sealing element 120 that extends across and closes the opening 111 in the base.

As can be seen from figure 2, there is a space or gap between the bottom of the bottle 101 and the outer cartridge casing 102. A sealing assembly is located in this space.

The sealing assembly comprises a helical or conical spring 122 located with its axis aligned with the long axis of the bottle 101 and the outer cartridge casing 102. The spring is formed so that tapers towards its lower end. A bung 124 is connected to the lower or outer end of the helical spring 122. The bung 124 is sized and shaped so that it can locate into the vent hole 110 to close and seal the vent hole 110 from inside the outer cartridge casing 102. The bung 124 has a rounded base, curving upwards and outwards, to assist with locating into the vent hole 110. The spring 122 pushes against the bung 124 from above so that it is pressed against the perimeter or rim of the vent hole 110 to close and seal the vent hole 110.

The bung 124 can be moved upwards against the spring 122 between a first, sealing position in which the bung 124 seals the vent hole 110, and a second, non-sealing position in which the bung 124 is lifted away from the vent hole 110 to allow the passage of gases/fluids through the vent hole. The helical spring 122 is configured to bias the sealing element 120 towards the first position.

In use, the cartridge assembly 110 is located within a liquid dispensing device such as an inhaler 10, the lower end or base 1000 of the inhaler 10 shown in figure 2.

The inhaler 10 is of the type where the base 1000 comprises a protruding member 14 on the inside-centre of the base, the protruding member 14 extending upwards from the base 100 towards the bung 124, and axially aligned with the helical spring 122 and the bung 124. The protruding member is configured so that in use it will press upwards against the bung 124 to push this away from the vent hole 110 and open the vent hole 110 when the inhaler is in certain configurations. This process is described in more detail below.

In use, once the cartridge assembly 110 is located in the inhaler 10, a dispensing tube (not shown) that forms part of the dispensing device 10 pierces the pierceable seal 104 and passes through the closure 103 into the collapsible bag 114. In use, when the liquid dispensing device 10 is actuated, the dispensing tube draws (by suction) a measure of liquid 116 out of the collapsible bag 114 for dispensing to a user. This suction action causes the collapsible bag 114 to collapse inwards and move upwards towards the top of the cartridge assembly 110 (i.e. towards the seal 104).

This reduction in the volume of the liquid 116 in the collapsible bag 114 creates a vacuum effect - the bag 114 is located inside the bottle 101, but is not connected or attached to the inner wall of the bottle 101. As the bag 114 collapses, this creates space between the outer surface of the bag, and the inner surface of the rigid wall of the bottle 101. If there is no equalising of the pressure between the interior of the bag and this space, then there is a pressure differential between this space and the interior of the bag - the vacuum effect - that makes it harder for the bag to continue to collapse with successive activations - the lower pressure outside the bag, in this space, 'sucks' the bag outwards to prevent is from collapsing. However, the container hole 113 allows gases to enter this space to equalise the pressure and to allow the bag to collapse. These gases are supplied to the interior of the outer cartridge casing 102 (and then to the interior of the bottle 101 via the container hole 113) via the vent hole 111 in the outer cartridge casing 102. If the vent hole 111 was simply an opening, effectively permanently open once the foil seal 120 is broken, then the thin-walled bag 114 would be directly exposed to atmosphere, and there would be significant evaporation through the wall of the bag 114, to atmosphere (via the container hole 113 and the vent hole 111). The bung 124 allows the equalisation of the pressure to allow the bag to collapse more easily, while also sealing the cartridge assembly 110 to help prevent evaporation.

The liquid delivery device 10 is further configured so that during actuation of the liquid dispensing device 10, the cartridge assembly 110 moves downwards within the liquid dispensing device. This movement causes the protruding member 14 to move against and to pierce the foil seal 120 (during the first actuation), and to engage with the bung 124 (first and all subsequent actuations) to move the bung 124 away from the vent hole 111. This movement of the bung 124 away from the vent hole 111 unseals the vent hole 111, allowing air external to the cartridge assembly 110 to enter the outer casing 102 of the cartridge through the vent hole 111. This then allows air to enter the bottle 101 through the container hole 113. This allows the pressure to be equalised inside the cartridge assembly 110.

At the end of the actuation, the cartridge assembly 110 moves upwards causing the bung 124 to disengage from the protruding member 14. The helical spring 122, which has been compressed during the first part of the actuation, then acts to push against the bung 124 to move the bung 124 back into engagement with the vent hole 111, sealing the vent hole 111. This re-sealing of the vent hole 111 helps to reduce the evaporation of liquid 116 from the collapsible bag 114 between actuations of the liquid dispensing device 10, thereby increasing the shelf life of the cartridge assembly 110.

### Cartridge - Second Embodiment

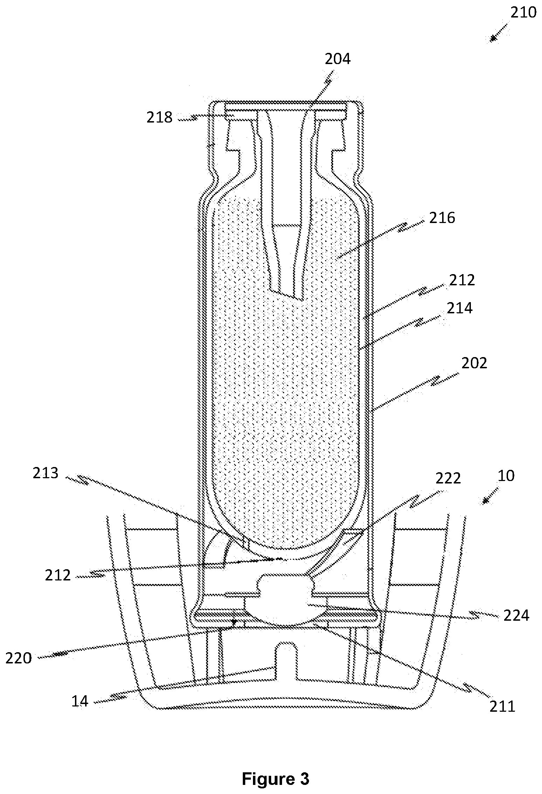

A second embodiment of cartridge or cartridge assembly is shown in figure 3.

In this embodiment, the outer cartridge casing 202 is the same as for the outer cartridge casing 102 of the first embodiment, having an opening or vent hole 211 that passes through the base or bottom of the outer cartridge casing 2, and a foil sealing element 220 that extends across and closes the opening 211 in the base.

The inner cartridge assembly 200 has the same outer appearance and profile as for the first embodiment, with the inner cartridge assembly 210 (the seal 204, the closure 203, the gasket 218) being substantially identical to the first embodiment. The bottle 201 is substantially identical at its top end to the bottle 101 of the first embodiment. However, the base or lower end of the bottle 201 is rounded or domed, with the collapsible bag 214 identical to the bag 114, but also following this shape at its base. A vent hole 213 is formed through the domed base of the bottle 201, allowing the passage of gases into the interior of the inner cartridge assembly.

Similarly to the first embodiment, there is a space or gap between the bottom of the bottle 101 and the outer cartridge casing 102, and a sealing assembly is located in this space.

The sealing assembly comprises a bung 224 that is biased against the vent hole 211 of the outer cartridge casing 202, by a finger disc spring 222 that also forms part of the sealing assembly. The bung 224 has a rounded base, curving upwards and outwards, to assist with locating into the vent hole 210. The finger disc spring 222 locates between the rounded or domed base of the bottle 201 and the upper end of the bung 224, and is configured so as to bias the sealing element bung 224 downwards to seal the vent hole 211.

In use, the cartridge is used with an inhaler 10, the same as for the cartridge of the first embodiment. As for the first embodiment, the protruding member 14 of the inhaler 10 extends upwards so that in use it will press upwards against the bung 224 to push this away from the vent hole 211 and open the vent hole 211 when the inhaler is actuated.

During the first actuation, the protruding member 14 moves against and pierces the foil seal 220, and engages with the bung 224 (first and all subsequent actuations) to move the bung 224 away from the vent hole 211. This movement of the bung 224 away from the vent hole 211 unseals the vent hole 211, allowing air external to the cartridge assembly 210 to enter the outer casing 202 of the cartridge through the vent hole 211. This then allows air to enter the bottle 201 through the container hole 113. This allows the pressure to be equalised inside the cartridge assembly 210.

At the end of the actuation, the cartridge assembly 210 moves upwards causing the bung 224 to disengage from the protruding member 14. The finger disc spring 222, which has been compressed during the first part of the actuation, then acts to push against the bung 224 to move the bung 224 back into engagement with the vent hole 211, re-sealing the vent hole 211. This re-sealing of the vent hole 211 helps to reduce the evaporation of liquid 216 from the collapsible bag 214 between actuations of the liquid dispensing device 10, thereby increasing the shelf life of the cartridge assembly 210.

### Cartridge - Third Embodiment

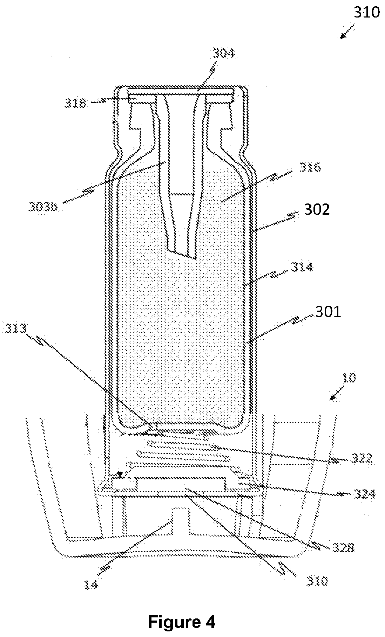

A third embodiment of cartridge or cartridge assembly 310 is shown in figure 4.

The cartridge assembly 310 in this embodiment has an outer cartridge casing 302 that is the same or substantially similar to the outer cartridge casings 102, 202 of the previous embodiments. The cartridge assembly 310 has an opening or vent hole 311 that passes through the base or bottom of the outer cartridge casing 302, and a foil sealing element 320 that extends across and closes the opening 311 in the base.

The inner cartridge assembly 300 is identical to the inner cartridge assembly 100 of the first embodiment.

Similarly to the first and second embodiments, there is a space or gap between the bottom of the bottle 301 and the outer cartridge casing 302, and a sealing assembly is located in this space.

The sealing assembly comprises a bung 324 that is biased against the vent hole 311 of the outer cartridge casing 302, by a helical spring 322 that also forms part of the sealing assembly. In this embodiment, the bung 324 comprises a main body portion that in plan view extends across substantially the entirety of the base of the outer cartridge casing 302, and an insert 328 that locates into a cavity in the lower face of the bung 324, the insert having a greater width or diameter than that of the vent hole 311. In the preferred embodiment, the insert 328 locates in the cavity so that it is flush with the lower surface of the main body portion of the bung. The main body portion is formed from a hard, rigid material that retains its shape when under compressive forces. The insert 328 is formed from a softer material that provides a good seal against the outer cartridge casing 302. The insert has a thickness of substantially 0.5mm in the preferred form, but can be thicker if required.

The helical spring 322 is configured to bias the bung 324 downwards to press the bung 324 against the lower inside surface of the outer cartridge casing 302. In this position, the insert 328 completely covers the vent hole 310, and forms a tight seal against the vent hole 310. It should be noted that in this embodiment, the helical spring 322 is downwardly tapered.

In use, the cartridge is used with an inhaler 10, the same as for the cartridge of the first embodiment. As for the first embodiment, the protruding member 14 of the inhaler 10 extends upwards so that in use it will press upwards against the bung 324 to push this away from the vent hole 311 and open the vent hole 311 when the inhaler is actuated.

## Claims

1. A cartridge for an inhaler, comprising:
an inner cartridge assembly (100) comprising a bottle and a collapsible bag (114), the bag (114) configured to contain a volume of liquid (116), the bottle (101) and bag (114) mutually configured so that the bag (114) is contained within the bottle (101), the bottle (101) further comprising a container hole (113) configured to allow gases to pass between the interior and exterior of the bottle;
an outer casing (102) configured to contain the inner cartridge assembly (100), the inner cartridge assembly (100) sealed within the outer casing (102), the outer casing (102) comprising a vent hole (111) passing through the casing from the exterior to the interior to allow gases to pass into the outer casing (102);
a sealing assembly (122, 124) configured to close and seal the vent hole (111);
the outer casing and inner cartridge assembly (100) further configured so that there is a gas path inside the cartridge between the container hole (113) and the vent hole (111);
the cartridge being **characterised in that** the sealing assembly (122, 124) has an open position that allows the passage of gases through the vent hole (111), and a closed position where the sealing assembly (122, 124) seals the vent hole (111), the sealing assembly (122, 124) being configured so that when the cartridge is used in an inhaler, actuating the inhaler temporarily opens the sealing assembly (112, 124) to allow the passage of gases therethrough.

2. A cartridge for an inhaler as claimed in claim 1 wherein the sealing assembly (122, 124) comprises a spring (122) and a bung (124), the bung and vent hole (111) mutually configured so that the bung (124) can locate onto the vent hole to seal the vent hole (111), the spring (122) configured to bias the bung (124) towards the vent hole (111).

3. A cartridge for an inhaler as claimed in claim 2 wherein the spring (122) comprises a conical spring.

4. A cartridge for an inhaler as claimed in claim 3 wherein the spring (122) is configured so as to taper towards the bung (124).

5. A cartridge for an inhaler as claimed in claim 2 wherein the spring (122) comprises a finger disc spring.

6. A cartridge for an inhaler as claimed in any one of claims 3 to 5 wherein the bung (124) comprises a rounded outer end.

7. A cartridge for an inhaler as claimed in claim 2 or claim 3 wherein the bung (124) comprises a main body portion and an insert that locates into a cavity in the main body portion, the insert having a greater width or diameter than that of the vent hole (111), the main body portion formed from a material that retains shape when subjected to compressive forces in use, the insert formed from a sealing material softer than the material used for the main body portion.

8. A cartridge for an inhaler as claimed in claim 7 wherein the spring (122) is configured so as to taper away from the bung (124).

9. A cartridge assembly (100) as claimed in any one of claims 1 to 8 further comprising a pierceable sealing element configured to extend across and close the vent hole (111).

10. **An inhaler** for delivery of liquid medicament to a user, the inhaler comprising:
a body containing the cartridge of any one of claims 1 to 9;
the body further comprising a protrusion configured to interact with the sealing assembly (122, 124) in use to temporarily open the sealing assembly (122, 124) during actuation of the inhaler.

11. An inhaler as claimed in claim 10 wherein the inhaler comprises a Soft Mist Inhaler.

## Patentansprüche

1. Kartusche für einen Inhalator, umfassend:
eine innere Kartuschenanordnung (100), die eine Flasche und einen zusammenfaltbaren Beutel (114) umfasst, wobei der Beutel (114) dazu eingerichtet ist, ein Flüssigkeitsvolumen (116) zu enthalten, wobei die Flasche (101) und der Beutel (114) derart gegenseitig eingerichtet sind, dass der Beutel (114) innerhalb der Flasche (101) enthalten ist, wobei die Flasche (101) ferner eine Behälteröffnung (113) umfasst, die dazu eingerichtet ist, Gase zwischen dem Inneren und dem Äußeren der Flasche durchtreten zu lassen;
ein Außengehäuse (102), das dazu eingerichtet ist, die innere Kartuschenanordnung (100) aufzunehmen, wobei die innere Kartuschenanordnung (100) innerhalb des Außengehäuses (102) abgedichtet ist, wobei das Außengehäuse (102) eine Entlüftungsöffnung (111) umfasst, die sich von dem Äußeren zu dem Inneren durch das Gehäuse hindurch erstreckt, um Gase in das Außengehäuse (102) eintreten zu lassen;
eine Dichtungsanordnung (122, 124), die dazu konfiguriert ist, die Entlüftungsöffnung (111) zu schließen und abzudichten;
wobei das Außengehäuse und die innere Kartuschenanordnung (100) ferner derart eingerichtet sind, dass innerhalb der Kartusche ein Gasweg zwischen der Behälteröffnung (113) und der Entlüftungsöffnung (111) vorhanden ist;
wobei die Kartusche **dadurch gekennzeichnet ist, dass** die Dichtungsanordnung (122, 124) eine Offenstellung aufweist, die den Durchtritt von Gasen durch die Entlüftungsöffnung (111) ermöglicht, und eine Schließstellung, in der die Dichtungsanordnung (122, 124) die Entlüftungsöffnung (111) abdichtet, wobei die Dichtungsanordnung (122, 124) derart eingerichtet ist, dass, wenn die Kartusche in einem Inhalator verwendet wird, ein Betätigen des Inhalators die Dichtungsanordnung (112, 124) vorübergehend öffnet, um den Durchtritt von Gasen dadurch zu ermöglichen.

2. Kartusche für einen Inhalator nach Anspruch 1, wobei die Dichtungsanordnung (122, 124) eine Feder (122) und einen Stopfen (124) umfasst, wobei der Stopfen und das Entlüftungsloch (111) gegenseitig so eingerichtet sind, dass der Stopfen (124) auf dem Entlüftungsloch angeordnet werden kann, um das Entlüftungsloch (111) abzudichten, wobei die Feder (122) so eingerichtet ist, dass sie den Stopfen (124) in Richtung des Entlüftungslochs (111) vorspannt.

3. Kartusche für einen Inhalator nach Anspruch 2, wobei die Feder (122) eine konische Feder umfasst.

4. Kartusche für einen Inhalator nach Anspruch 3, wobei die Feder (122) dazu eingerichtet ist, sich zu dem Stopfen (124) hin zu verjüngen.

5. Kartusche für einen Inhalator nach Anspruch 2, wobei die Feder (122) eine Fingerfederscheibe umfasst.

6. Kartusche für einen Inhalator nach einem der Ansprüche 3 bis 5, wobei der Stopfen (124) ein abgerundetes äußeres Ende umfasst.

7. Kartusche für einen Inhalator nach Anspruch 2 oder 3, bei der der Stopfen (124) einen Hauptkörperabschnitt und einen Einsatz aufweist, der in einem Hohlraum in dem Hauptkörperabschnitt angeordnet ist, wobei der Einsatz eine größere Breite oder einen größeren Durchmesser als die des Entlüftungslochs (111) aufweist, wobei der Hauptkörperabschnitt aus einem Material gebildet ist, das die Form beibehält, wenn er im Gebrauch Druckkräften ausgesetzt wird, wobei der Einsatz aus einem Dichtungsmaterial gebildet ist, das weicher ist als das Material, das für den Hauptkörperabschnitt verwendet wird.

8. Kartusche für einen Inhalator nach Anspruch 7, wobei die Feder (122) dazu eingerichtet ist, sich von dem Stopfen (124) weg zu verjüngen.

9. Kartuschenanordnung (100) nach einem der Ansprüche 1 bis 8, ferner umfassend ein durchstechbares Dichtungselement, das dazu konfiguriert ist, sich über die Entlüftungsöffnung (111) hinweg zu erstrecken und diese zu verschließen.

10. Inhalator zur Abgabe eines flüssigen Medikaments an einen Benutzer, wobei der Inhalator umfasst:
einen Körper, der die Kartusche nach einem der Ansprüche 1 bis 9 enthält;
wobei der Körper ferner einen Vorsprung umfasst, der dazu eingerichtet ist, im Gebrauch mit der Dichtungsanordnung (122, 124) zusammenzuwirken, um die Dichtungsanordnung (122, 124) während einer Betätigung des Inhalators vorübergehend zu öffnen.

11. Inhalator nach Anspruch 10, wobei der Inhalator einen Soft-Mist-Inhaler umfasst.

## Revendications

1. Cartouche pour un inhalateur, comprenant :
un ensemble cartouche interne (100) comprenant une bouteille et un sac repliable (114), le sac (114) étant configuré pour contenir un volume de liquide (116), la bouteille (101) et le sac (114) étant mutuellement configurés de sorte que le sac (114) est contenu au sein de la bouteille (101), la bouteille (101) comprenant en outre un trou de conteneur (113) configuré pour permettre aux gaz de passer entre l'intérieur et l'extérieur de la bouteille ;
un boîtier externe (102) configuré pour contenir l'ensemble cartouche interne (100), l'ensemble cartouche interne (100) étant scellé au sein du boîtier externe (102), le boîtier externe (102) comprenant un trou d'évent (111) passant à travers le boîtier depuis l'extérieur vers l'intérieur pour permettre aux gaz de passer jusque dans le boîtier externe (102) ;
un ensemble d'étanchéité (122, 124) configuré pour fermer et rendre étanche le trou d'évent (111) ;
le boîtier externe et l'ensemble cartouche interne (100) sont en outre configurés de sorte qu'il existe un trajet de gaz à l'intérieur de la cartouche entre le trou de conteneur (113) et le trou d'évent (111) ;
la cartouche étant **caractérisée en ce que** l'ensemble d'étanchéité (122, 124) a une position ouverte qui permet le passage de gaz à travers le trou d'évent (111), et une position fermée où l'ensemble d'étanchéité (122, 124) rend étanche le trou d'évent (111), l'ensemble d'étanchéité (122, 124) étant configuré de sorte que lorsque la cartouche est utilisée dans un inhalateur, l'actionnement de l'inhalateur ouvre temporairement l'ensemble d'étanchéité (112, 124) pour permettre le passage de gaz à travers celui-ci.

2. Cartouche pour un inhalateur selon la revendication 1, dans laquelle l'ensemble d'étanchéité (122, 124) comprend un ressort (122) et un bouchon (124), le bouchon et le trou d'évent (111) étant mutuellement configurés de telle sorte que le bouchon (124) peut se placer sur le trou d'évent pour rendre étanche le trou d'évent (111), le ressort (122) étant configuré pour solliciter le bouchon (124) vers le trou d'évent (111).

3. Cartouche pour un inhalateur selon la revendication 2, dans laquelle le ressort (122) comprend un ressort conique.

4. Cartouche pour un inhalateur selon la revendication 3, dans laquelle le ressort (122) est configuré de manière à se rétrécir vers le bouchon (124).

5. Cartouche pour un inhalateur selon la revendication 2, dans laquelle le ressort (122) comprend un ressort à disque à doigts.

6. Cartouche pour un inhalateur selon l'une quelconque des revendications 3 à 5, dans laquelle le bouchon (124) comprend une extrémité externe arrondie.

7. Cartouche pour un inhalateur selon la revendication 2 ou la revendication 3, dans laquelle le bouchon (124) comprend une portion corps principal et un insert qui se place dans une cavité dans la portion corps principal, l'insert ayant une largeur ou un diamètre plus grand que celui du trou d'évent (111), la portion corps principal étant formée à partir d'un matériau qui conserve sa forme lorsqu'il est soumis à des forces de compression en utilisation, l'insert étant formé à partir d'un matériau d'étanchéité plus mou que le matériau utilisé pour la portion corps principal.

8. Cartouche pour un inhalateur selon la revendication 7, dans laquelle le ressort (122) est configuré de manière à se rétrécir en s'éloignant du bouchon (124).

9. Ensemble cartouche (100) selon l'une quelconque des revendications 1 à 8, comprenant en outre un élément d'étanchéité perçable configuré pour s'étendre en travers du trou d'évent (111) et le fermer.

10. Inhalateur pour l'administration d'un médicament liquide à un utilisateur, l'inhalateur comprenant :
un corps contenant la cartouche selon l'une quelconque des revendications 1 à 9 ;
le corps comprenant en outre une saillie configurée pour interagir avec l'ensemble d'étanchéité (122, 124) lors de l'utilisation pour ouvrir temporairement l'ensemble d'étanchéité (122, 124) pendant l'actionnement de l'inhalateur.

11. Inhalateur selon la revendication 10, dans lequel l'inhalateur comprend un inhalateur de brume ultra fine (Soft Mist).
